# EUROPEAN PATENT SPECIFICATION

(11) **EP 1 973 398 B1**
(45) Date of publication and mention of the grant of the patent: **06.01.2016**
(21) Application number: 06717596.8
(22) Date of filing: 06.01.2006
(51) Int. Cl.: A01N 25/00, A01N 65/00, A61P 35/00, A61K 31/6615

(54) **USE OF INOSITOL-TRIPYROPHOSPHATE IN TREATING TUMORS MEDIATED BY ANGIOGENESIS**
VERWENDUNG VON INOSIT-TRIPYROPHOSPHAT BEI DER BEHANDLUNG VON ANGIOGENESEVERMITTELTEN TUMOREN
UTILISATION D'INOSITOL-TRIPYROPHOSPHATE DANS LE TRAITEMENT DE TUMEURS MÉDIÉES PAR L'ANGIOGÉNÈSE

(43) Date of publication of application: 01.10.2008
(73) Proprietor: NormOxys, Inc., Boston, MA 02135 (US)
(72) Inventor: NICOLAU, Claude, Newton, Massachusetts 02459 (US); GREFERATH, Ruth, 77694 Kehl (DE); FYLAKTAKIDOU, Konstantina, C., GR-55438 Thessaloniki (GR); LEHN, Jean-marie, F-67000 Strasbourg (FR)
(74) Representative: Grund, Martin
(86) International application number: PCT/US2006/000420
(87) International publication number: WO 2007/081315

(56) References cited:
- WO-A1-03/092700
- WO-A1-2006/102060
- US-A- 4 952 717
- US-A1- 2005 250 743
- FYLAKTAKIDOU K C ET AL: "Inositol tripyrophosphate: a new membrane permeant allosteric effector of haemoglobin" 15 March 2005 (2005-03-15), BIOORGANIC & MEDICINAL CHEMISTRY LETTERS, PERGAMON, ELSEVIER SCIENCE, GB LNKD- DOI:10.1016/J.BMCL.2005.01.064, PAGE(S) 1605 - 1608 , XP025313553 ISSN: 0960-894X [retrieved on 2005-03-15] * figure 1 * * page 1608, column 1, paragraph 3 *
- JOHNSON L F ET AL: "Structure of phytic acids" 1 January 1969 (1969-01-01), CANADIAN JOURNAL OF CHEMISTRY, NRC RESEARCH PRESS, CA LNKD- DOI:10.1139/V69-008, PAGE(S) 63 - 73 , XP009122097 ISSN: 0008-4042 * abstract *
- LUO, HONGBO R. ET AL: "Inositol pyrophosphates mediate chemotaxis in Dictyostelium via pleckstrin homology domain-PtdIns(3,4,5)P3 interactions" CELL (CAMBRIDGE, MA, UNITED STATES) , 114(5), 559-572 CODEN: CELLB5; ISSN: 0092-8674, 2003, XP8121659
- J. BIOL. CHEM., vol. 276, 2001, pages 24965-24970, XP8121763
- FYLAKTAKIDOU K.C. ET AL.: 'Inositol tripyrophosphate: a new membrane permeant allosteric effector of haemoglobin' BIOORG. MED. CHEM. LETT. vol. 15, no. 6, 2005, pages 1605 - 1608, XP004771154
- AMORINO G.P. ET AL.: 'Enhancement of Tumor oxygenation and Radiation Response by the Allosteric Effector of hemoglobin, RSR13' RADIAT. RES. vol. 156, 2001, pages 294 - 300, XP008083599
- TEICHER B.A. ET AL.: 'Allosteric Effectors of hemoglobin as Modulators of chemotherapy and radiation therapy in vitro and in vivo' CANCER CHEMOTHER. PHARMACOL. vol. 42, no. 1, 1998, pages 24 - 30, XP003015978
- PAGEL P.S. ET AL.: 'RSR13, a Synthetic Modifer of Hemoglobin-Oxygen Affinity, enhances the Recovery of Stunned Myocardium in Anestetized Dogs' J. OF PHARM. EXP. THER. vol. 285, no. 1, 1998, pages 1 - 15, XP003015979
- KILGORE K.S. ET AL.: 'RSR13, a Synthetic allosteric modifier of hemoglobin, Improves Myocardial recovery Following Hypothermic Cardiopulmonary Bypass' CIRCULATION vol. 100, 1999, pages II-351 - II-356, XP003015980
- VUCENIK I. ET AL.: 'Anti-angiogenic activity of inositol hexaphosphate (IP6)' CARCINOGENESIS vol. 25, no. 11, 2004, pages 2115 - 2123, XP003015981
- KIEDA ET AL.: "Suppression of hypoxia induced...", PNAS, 2006,

## Description

### FIELD OF THE INVENTION

Disclosed are compositions for using inositol-tripyrophosphate (ITPP) to treat blood borne tumors, solid tumors and tumor metastasis ITPP is an allosteric effector of hemoglobin which has the ability to cross the plasma membrane of red blood cells and lower the oxygen affinity of the hemoglobin of red blood cells. The present invention is further directed to the use of ITPP to treat the claimed disorders wherein the tumors are associated to abnormal angiogenesis and wherein the tumors are hypoxic tumors. Disclosed is further the use of ITPP to enhance PO₂ in hypoxic tumors.

### BACKGROUND OF THE INVENTION

In the vascular system of an adult human being, blood has a volume of about 5 to 6 liters. Approximately one half of this volume is occupied by cells, including red blood cells (erythrocytes), white blood cells (leukocytes), and blood platelets. Red blood cells comprise the majority of the cellular components of blood. Plasma, the liquid portion of blood, is approximately 90 percent water and 10 percent various solutes. These solutes include plasma proteins, organic metabolites and waste products, and inorganic compounds.

The major function of red blood cells is to transport oxygen from the lungs to the tissues of the body, and transport carbon dioxide from the tissues to the lungs for removal. Very little oxygen is transported by the blood plasma because oxygen is only sparingly soluble in aqueous solutions. Most of the oxygen carried by the blood is transported by the hemoglobin of the erythrocytes. Erythrocytes in mammals do not contain nuclei, mitochondria or any other intracellular organelles, and they do not use oxygen in their own metabolism. Red blood cells contain about 35 percent by weight hemoglobin, which is responsible for binding and transporting oxygen.

Hemoglobin is a protein having a molecular weight of approximately 64,500 daltons. It contains four polypeptide chains and four heme prosthetic groups in which iron atoms are bound in the ferrous state. Normal globin, the protein portion of the hemoglobin molecule, consists of two alpha chains and two beta chains. Each of the four chains has a characteristic tertiary structure in which the chain is folded. The four polypeptide chains fit together in an approximately tetrahedral arrangement, to constitute the characteristic quaternary structure of hemoglobin. There is one heme group bound to each polypeptide chain which can reversibly bind one molecule of molecular oxygen. When hemoglobin combines with oxygen, oxyhemoglobin is formed. When oxygen is released, the oxyhemoglobin is reduced to deoxyhemoglobin.

Delivery of oxygen to tissues, including tumors, depends upon a number of factors including, but not limited to, the volume of blood flow, the number of red blood cells, the concentration of hemoglobin in the red blood cells, the oxygen affinity of the hemoglobin and, in certain species, on the molar ratio of intraerythrocytic hemoglobins with high and low oxygen affinity. The oxygen affinity of hemoglobin depends on four factors as well, namely: (1) the partial pressure of oxygen; (2) the pH; (3) the concentration of 2,3-diphosphoglycerate (DPG) in the hemoglobin; and (4) the concentration of carbon dioxide. In the lungs, at an oxygen partial pressure of 100 mm Hg, approximately 98% of circulating hemoglobin is saturated with oxygen. This represents the total oxygen transport capacity of the blood. When fully oxygenated, 100 ml of whole mammalian blood can carry about 21 ml of gaseous oxygen.

The effect of the partial pressure of oxygen and the pH on the ability of hemoglobin to bind oxygen is best illustrated by examination of the oxygen saturation curve of hemoglobin. An oxygen saturation curve plots the percentage of total oxygen-binding sites of a hemoglobin molecule that are occupied by oxygen molecules when solutions of the hemoglobin molecule are in equilibrium with different partial pressures of oxygen in the gas phase.

The oxygen saturation curve for hemoglobin is sigmoid. Thus, binding the first molecule of oxygen increases the affinity of the remaining hemoglobin for binding additional oxygen molecules. As the partial pressure of oxygen is increased, a plateau is approached at which each of the hemoglobin molecules is saturated and contains the upper limit of four molecules of oxygen.

The reversible binding of oxygen by hemoglobin is accompanied by the release of protons, according to the equation:

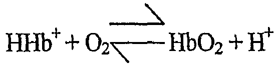

Thus, an increase in the pH will pull the equilibrium to the right and cause hemoglobin to bind more oxygen at a given partial pressure. A decrease in the pH will decrease the amount of oxygen bound.

In the lungs, the partial pressure of oxygen in the air spaces is approximately 90 to 100 mm Hg and the pH is also high relative to normal blood pH (up to 7.6). Therefore, hemoglobin will tend to become almost maximally saturated with oxygen in the lungs. At that pressure and pH, hemoglobin is approximately 98 percent saturated with oxygen. On the other hand, in the capillaries in the interior of the peripheral tissues, the partial pressure of oxygen is only about 25 to 40 mm Hg and the pH is also nearly neutral (about 7.2 to 7.3). Because muscle cells use oxygen at a high rate, thereby lowering the local concentration of oxygen, the release of some of the bound oxygen to the tissue is favored. As the blood passes through the capillaries in the muscles, oxygen will be released from the nearly saturated hemoglobin in the red blood cells into the blood plasma and thence into the muscle cells. Hemoglobin will release about a fourth of its bound oxygen as it passes through the muscle capillaries, so that when it leaves the muscle, it will be only about 75 percent saturated. In general, the hemoglobin in the venous blood leaving the tissue cycles between about 65 and 97 percent saturation with oxygen in its repeated circuits between the lungs and the peripheral tissues. Thus, oxygen partial pressure and pH function together to effect the release of oxygen by hemoglobin.

A third important factor in regulating the degree of oxygenation of hemoglobin is the allosteric effector 2,3-diphosphoglycerate (DPG). DPG is the normal physiological effector of hemoglobin in mammalian erythrocytes. DPG regulates the oxygen-binding affinity of hemoglobin in the red blood cells in relationship to the oxygen partial pressure in the lungs. The higher the concentration of DPG in the cell, the lower the affinity of hemoglobin for oxygen.

When the delivery of oxygen to the tissues is chronically reduced, the concentration of DPG in the erythrocytes is higher than in normal individuals. For example, at high altitudes the partial pressure of oxygen is significantly less. Correspondingly, the partial pressure of oxygen in the tissues is less. Within a few hours after a normal human subject moves to a higher altitude, the DPG level in the red blood cells increases, causing more DPG to be bound and the oxygen affinity of the hemoglobin to decrease. Increases in the DPG level of red cells also occur in patients suffering from hypoxia. This adjustment allows the hemoglobin to release its bound oxygen more readily to the tissues to compensate for the decreased oxygenation of hemoglobin in the lungs. The reverse change occurs when people are acclimated to high altitudes and descend to lower altitudes.

As normally isolated from blood, hemoglobin contains a considerable amount of DPG. When hemoglobin is "stripped" of its DPG, it shows a much higher affinity for oxygen. When DPG is increased, the oxygen binding affinity of hemoglobin decreases. A physiologic allosteric effector such as DPG is therefore essential for the normal release of oxygen from hemoglobin in the tissues.

While DPG is the normal physiologic effector of hemoglobin in mammalian red blood cells, phosphorylated inositols are found to play the same role in the erythrocytes of some birds and reptiles. Although inositol hexaphosphate (IHP) is unable to pass through the mammalian erythrocyte membrane, it is capable of combining with hemoglobin of mammalian red blood cells at the binding site of DPG to modify the allosteric conformation of hemoglobin, the effect of which is to reduce the affinity of hemoglobin for oxygen. For example, DPG can be replaced by IHP, which is far more potent than DPG in reducing the oxygen affinity of hemoglobin. IHP has a 1000-fold higher affinity to hemoglobin than DPG (R. E. Benesch et al., Biochemistry, Vol. 16, pages 2594-2597(1977)) and increases the P₅₀ of hemoglobin up to values of 96.4 mm, Hg at pH 7.4, and 37 degrees C. (J. Biol. Chem., Vol. 250, pages 7093-7098(1975)).

The oxygen release capacity of mammalian red blood cells can be enhanced by introducing certain allosteric effectors of hemoglobin into erythrocytes, thereby decreasing the affinity of hemoglobin for oxygen and improving the oxygen economy of the blood. This phenomenon suggests various medical applications for treating individuals who are experiencing lowered oxygenation of their tissues due to the inadequate function of their lungs or circulatory system.

Because of the potential medical benefits to be achieved from the use of these modified erythrocytes, various techniques have been developed in the prior art to enable the encapsulation of allosteric effectors of hemoglobin in erythrocytes. Accordingly, numerous devices have been designed to assist or simplify the encapsulation procedure. The encapsulation methods known in the art include osmotic pulse (swelling) and reconstitution of cells, controlled lysis and resealing, incorporation of liposomes, and electroporation. Current methods of electroporation make the procedure commercially impractical on a scale suitable for commercial use.

The following references describe the incorporation of polyphosphates into red blood cells by the interaction of liposomes loaded with IHP: Gersonde, et al., "Modification of the Oxygen Affinity of Intracellular Haemoglobin by Incorporation of Polyphosphates into Intact Red Blood Cells and Enhanced 02 Release in the Capillary System", Biblthca. Haemat., No. 46, pp. 81-92(1980); Gersonde, et al., "Enhancement of the O2 Release Capacity and of the Bohr-Effect of Human Red Blood Cells after Incorporation of Inositol Hexaphosphate by Fusion with Effector-Containing Lipid Vesicles", Origins of Cooperative Binding of Hemoglobin, (1982); and Weiner, "Right Shifting of Hb-O2 Dissociation in Viable Red Cells by Liposomal Technique," Biology of the Cell, Vol. 47, (1983).

Additionally, U.S. Pat. Nos. 4,192,869, 4,321,259, and 4,473,563 to Nicolau et al. describe a method whereby fluid-charged lipid vesicles are fused with erythrocyte membranes, depositing their contents into the red blood cells. In this manner it is possible to transport allosteric effectors such as IHP into erythrocytes, where, due to its much higher binding constant IHP replaces DPG at its binding site in hemoglobin.

In accordance with the liposome technique, IHP is dissolved in a phosphate buffer until the solution is saturated and a mixture of lipid vesicles is suspended in the solution. The suspension is then subjected to ultrasonic treatment or an injection process, and then centrifuged. The upper suspension contains small lipid vesicles containing IHP, which are then collected. Erythrocytes are added to the collected suspension and incubated, during which time the lipid vesicles containing IHP fuse with the cell membranes of the erythrocytes, thereby depositing their contents into the interior of the erythrocyte. The modified erythrocytes are then washed and added to plasma to complete the product.

The drawbacks associated with the liposomal technique include poor reproducibility of the IHP concentrations incorporated in the red blood cells and significant hemolysis of the red blood cells following treatment. Additionally, commercialization is not practical because the procedure is tedious and complicated.

In an attempt to solve the drawbacks associated with the liposomal technique, a method of lysing and the resealing red blood cells was developed. This method is described in the following publication: Nicolau, et al., "Incorporation of Allosteric Effectors of Hemoglobin in Red Blood Cells. Physiologic Effects," Biblthca. Haemat., No. 51, pp. 92-107, (1985). Related U.S. Pat. Nos. 4,752,586 and 4,652,449 to Ropars et al. also describe a procedure of encapsulating substances having biological activity in human or animal erythrocytes by controlled lysis and resealing of the erythrocytes, which avoids the red blood cell-liposome interactions.

The technique is best characterized as a continuous flow dialysis system which functions in a manner similar to the osmotic pulse technique. Specifically, the primary compartment of at least one dialysis element is continuously supplied with an aqueous suspension of erythrocytes while the secondary compartment of the dialysis element contains an aqueous solution which is hypotonic with respect to the erythrocyte suspension. The hypotonic solution causes the erythrocytes to lyse. The erythrocyte lysate is then contacted with the biologically active substance to be incorporated into the erythrocyte. To reseal the membranes of the erythrocytes, the osmotic and/or oncotic pressure of the erythrocyte lysate is increased and the suspension of resealed erythrocytes is recovered.

In related U.S. Pat. Nos. 4,874,690 and 5,043,261 to Goodrich et al., a related technique involving lyophilization and reconstitution of red blood cells is disclosed. As part of the process of reconstituting the red blood cells, the addition of various polyanions, including IHP, is described. Treatment of the red blood cells according to the process disclosed results in a cell with unaffected activity. Presumably, the IHP is incorporated into the cell during the reconstitution process, thereby maintaining the activity of the hemoglobin.

In U.S. Pat. Nos. 4,478,824 and 4,931,276 to Franco et al., a second related method and apparatus is described for introducing effectively non-ionic agents, including IHP, into mammalian red blood cells by effectively lysing and resealing the cells. The procedure is described as the "osmotic pulse technique." In practicing the osmotic pulse technique, a supply of packed red blood cells is suspended and incubated in a solution containing a compound which readily diffuses into and out of the cells, the concentration of the compound being sufficient to cause diffusion thereof into the cells so that the contents of the cells become hypertonic. Next, a transmembrane ionic gradient is created by diluting the solution containing the hypertonic cells with an essentially isotonic aqueous medium in the presence of at least one desired agent to be introduced, thereby causing diffusion of water into the cells with a consequent swelling and an increases in permeability of the outer membranes of the cells. This "osmotic pulse" causes the diffusion of water into the cells and a resultant swelling of the cells which increase the permeability of the outer cell membrane to the desired agent. The increase in permeability of the membrane is maintained for a period of time sufficient only to permit transport of least one agent into the cells and diffusion of the compound out of the cells.

Polyanions which may be used in practicing the osmotic pulse technique include pyrophosphate, tripolyphosphate, phosphorylated inositols, 2,3-diphosphoglycerate (DPG), adenosine triphosphate, heparin, and polycarboxylic acids which are watersoluble, and non-disruptive to the lipid outer bilayer membranes of red blood cells.

The osmotic pulse technique has several shortcomings including low yield of encapsulation, incomplete resealing, lose of cell content and a corresponding decrease in the life span of the cells. The technique is tedious, complicated and unsuited to automation. For these reasons, the osmotic pulse technique has had little commercial success.

Another method for encapsulating various biologically-active substances in erythrocytes is electroporation. Electroporation has been used for encapsulation of foreign molecules in different cell types, including IHP in red blood cells, as described in Mouneimne, et al., "Stable rightward shifts of the oxyhemoglobin dissociation curve induced by encapsulation of inositol hexaphosphate in red blood cells using electroporation," FEBS, Vol. 275, No. 1, 2, pp. 117-120 (1990) Also see U.S. Patent No. 5,612,207.

Angiogenesis is the generation of new blood vessels into a tissue or organ and is related to oxygen tension in the tissues. Under normal physiological conditions, humans and animals undergo angiogenesis only in very specific, restricted situations. For example, angiogenesis is normally observed in wound healing, fetal and embryonal development, and formation of the corpus luteum, endometrium and placenta.

Angiogenesis is controlled through a highly regulated system of angiogenic stimulators and inhibitors. The control of angiogenesis is altered in certain disease states and, in many cases, pathological damage associated with the diseases is related to uncontrolled angiogenesis. Both controlled and uncontrolled angiogenesis are thought to proceed in a similar manner. Endothelial cells and pericytes, surrounded by a basement membrane, form capillary blood vessels. Angiogenesis begins with the erosion of the basement membrane by enzymes released by endothelial cells and leukocytes. Endothelial cells, lining the lumen of blood vessels, then protrude through the basement membrane. Angiogenic stimulants induce the endothelial cells to migrate through the eroded basement membrane. The migrating cells form a "sprout" off the parent blood vessel where the endothelial cells undergo mitosis and proliferate. The endothelial sprouts merge with each other to form capillary loops, creating a new blood vessel.

Persistent, unregulated angiogenesis occurs in many disease states, tumor metastases, and abnormal growth by endothelial cells. The diverse pathological disease states in which unregulated angiogenesis is present have been grouped together as angiogenic-dependent or angiogenic-associated diseases.

The hypothesis that tumor growth is angiogenesis-dependent was first proposed in 1971. (Folkman, New Eng. J. Med., 285:1182-86 (1971)). In its simplest terms, this hypothesis states: "Once tumor 'take' has occurred, every increase in tumor cell population must be preceded by an increase in new capillaries converging on the tumor." Tumor 'take' is currently understood to indicate a prevascular phase of tumor growth in which a population of tumor cells occupying a few cubic millimeters volume, and not exceeding a few million cells, can survive on existing host microvessels. Expansion of tumor volume beyond this phase requires the induction of new capillary blood vessels. For example, pulmonary micrometastases in the early prevascular phase in mice would be undetectable except by high power microscopy on histological sections.

Angiogenesis has been associated with a number of different types of cancer, including solid tumors and blood-borne tumors. Solid tumors with which angiogenesis has been associated include, but are not limited to, rhabdomyosarcomas, retinoblastoma, Ewing's sarcoma, neuroblastoma, and osteosarcoma. Angiogenesis is also associated with blood-borne tumors, such as leukemias, any of various acute or chronic neoplastic diseases of the bone marrow in which unrestrained proliferation of white blood cells occurs, usually accompanied by anemia, impaired blood clotting, and enlargement of the lymph nodes, liver and spleen. It is believed that angiogenesis plays a role in the abnormalities in the bone marrow that give rise to leukemia tumors and multiple myeloma diseases.

One of the most frequent angiogenic diseases of childhood is the hemangioma. A hemangioma is a tumor composed of newly formed blood vessels. In most cases the tumors are benign and regress without intervention. In more severe cases, the tumors progress to large cavernous and infiltrative forms and create clinical complications. Systemic forms of hemangiomas, hemangiomatoses, have a high mortality rate. Therapy-resistant hemangiomas exist that cannot be treated with therapeutics currently in use.

Another angiogenesis associated disease is rheumatoid arthritis. The blood vessels in the synovial lining of the joints undergo angiogenesis. In addition to forming new vascular networks, the endothelial cells release factors and reactive oxygen species that lead to pannus growth and cartilage destruction. Angiogenesis may also play a role in osteoarthritis. The activation of the chondrocytes by angiogenic-related factors contributes to the destruction of the joint. At a later stage, the angiogenic factors promote new bone growth. Therapeutic intervention that prevents the cartilage destruction could halt the progress of the disease and provide relief for persons suffering with arthritis.

Chronic inflammation may also involve pathological angiogenesis. Such diseases as ulcerative colitis and Crohn's disease show histological changes with the ingrowth of new blood vessels into inflamed tissues. Bartonelosis, a bacterial infection found in South America, can result in a chronic stage that is characterized by proliferation of vascular endothelial cells. Another pathological role associated with angiogenesis is found in atherosclerosis. The plaques formed within the lumen of blood vessels have been shown to have angiogenic stimulatory activity.

As mentioned above, several lines of evidence indicate that angiogenesis is essential for the growth and persistence of solid tumors and their metastases. Once angiogenesis is stimulated, tumors upregulate the production of a variety of angiogenic factors, including fibroblast growth factors (aFGF and bFGF) and vascular endothelial growth factor/vascular permeability factor (VEGF/VPF) [2,3].

The role of VEGF in the regulation of angiogenesis has been the object of intense investigation [5-10]. Whereas VEGF represents a critical, rate-limiting step in physiological angiogenesis, it appears to be also important in pathological angiogenesis, such as that associated with tumor growth [11]. VEGF is also known as vascular permeability factor, based on its ability to induce vascular leakage [13]. Several solid tumors produce ample amounts of VEGF, which stimulates proliferation and migration of endothelial cells, thereby inducing neovascularization [12,13]. VEGF expression has been shown to significantly affect the prognosis of different kinds of human cancer. Oxygen tension in the tumor has a key role in regulating the expression of VEGF gene. VEGF mRNA expression is induced by exposure to low oxygen tension under a variety of pathophysiological circumstances [13]. Growing tumors are characterized by hypoxia, which induces expression of VEGF and may also be a predictive factor for the occurrence of metastatic disease.

What is needed, therefore, is a composition that can regulate oxygen tension in the tissue, especially a tumor. In addition, what is needed is a simple and easily administered, preferably orally, composition that is capable of causing significant right shifts of the P50 value for red blood cells.

### SUMMARY OF THE INVENTION

The invention is defined by the appended claims.

The present invention provides a composition comprising inositol-tripyrophosphate (ITPP) that is effective in treating diseases characterized by abnormal angiogenesis. Also disclosed are methods of using ITPP for increasing the regulated delivery of oxygen to tissues including tumors. For example, the regulation of vascular endothelial growth factor (VEGF) in a human or animal can be effected using ITPP which has entered the red blood cell, thus lowering the affinity for oxygen of circulating erythrocytes. The effect of ITPP on VEGF mRNA expression, protein concentration, and tumor cell proliferation are possible with the present invention. Also, a method of regulating VEGF expression, both in vitro and in vivo, has been developed using ITPP.

Further disclosed are compositions and methods for using ITPP in red blood cells to deliver oxygen to solid tumors, to inhibit angiogenesis and to enhance radiation sensitivity of hypoxic tumors. Further disclosed is the use of ITPP to enhance PO₂ in hypoxic tumors. ITPP, being an allosteric effector of hemoglobin, is capable of reducing hemoglobin's affinity for oxygen and enhances the release of oxygen by hemoglobin. Upon cellular demand, ITPP inhibits VEGF expression in tumor cells and, thus, angiogenesis.

Disclosed is the treatment of diseases characterized by undesirable angiogenesis or undesirable angiogenesis, including, but is not limited to, excessive or abnormal stimulation of endothelial cells (e.g. atherosclerosis), blood borne tumors, solid tumors and tumor metastasis, benign tumors, for example, hemangiomas, acoustic neuromas, neurofibromas, trachomas, and pyogenic granulomas, vascular malfunctions, abnormal wound healing, inflammatory and immune disorders, Bechet's disease, gout, or gouty arthritis, diabetic retinopathy and other ocular angiogenic diseases such as retinopathy of prematurity (retrolental fibroplasic), macular degeneration, corneal graft rejection, neovascular glaucoma and Osier Weber syndrome (Osler-Weber-Rendu disease). Cancers that can be treated by the present invention include, but is not limited to, breast cancer, prostrate cancer, renal cell cancer, brain cancer, ovarian cancer, colon cancer, bladder cancer, pancreatic cancer, stomach cancer, esophageal cancer, cutaneous melanoma, liver cancer, lung cancer, testicular cancer, kidney cancer, bladder cancer, cervical cancer, lymphoma, parathyroid cancer, penile cancer, rectal cancer, small intestine cancer, thyroid cancer, uterine cancer, Hodgkin's lymphoma, lip and oral cancer, skin cancer, leukemia or multiple myeloma.

Disclosed is a composition and method for treating cancer and other angiogenic disease states and conditions.

Disclosed is a composition and method for enhancing oxygen delivery to hypoxic tumors.

Disclosed is a composition and method for inhibiting angiogenesis. A further object of the invention is to provide a composition and method for enhancing radiation sensitivity of hypoxic tumors.

Disclosed is a composition and method of treating hypoxic tumors and diseases.

Disclosed is a composition and method that can regulate oxygen tension in the tissue, especially a tumor.

Disclosed is a simple and easily administered, preferably orally, composition that is capable of causing significant right shifts of the P50 value for red blood cells.

These and other objects, features and advantages of the present disclosure will become apparent after a review of the following detailed description of the disclosed embodiments.

### BRIEF DESCRIPTION OF THE DRAWINGS

Figure 1 depicts the chemical structure of inositol-tri-pyrophosphate (ITPP).
Figure 2 shows the time course of the induced right shift of the O₂-hemoglobin dissociation curve (ODC) in the mice ingesting ITPP for 4 days as well as the absence of significant P50 shifts in the control animals.
Figure 3 shows that the level of ions, such as sodium and potassium and calcium, were normal after oral application of ITPP in mice.
Figure 4 shows the relation of P50 shift [%] to number of erythrocytes/mm³ in mice having received ITPP.
Figure 5 demonstrates ITPP toleration by mice, up to a concentration of 150mM. The level of ions, such as sodium, potassium and calcium were normal after intraperitoneal (ip) injection.
Figure 6 shows an agarose gel indicating the VEGF mRNA concentrations in tumors from control and ITPP drinking animals.
Figure 7 shows the Western blot assay of the expressed VEGF in tumors of control and ITPP-treated Lewis Lung carcinoma (LLC) tumor-bearing animals.

### DETAILED DESCRIPTION OF THE INVENTION

The invention is defined by the appended claims.

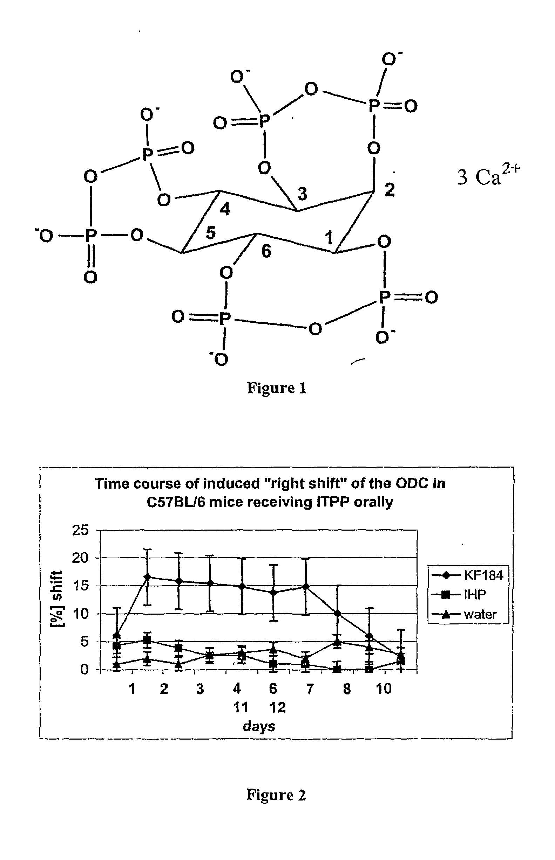

Compositions that are useful in accordance with the present invention include inositol-tripyrophosphate (ITPP) (Figure 1) and salts thereof like Na, Ca, NH4 salts of

ITPP, which exhibits anti-angiogenic and anti-tumor properties, and are useful in controlling angiogenesis-, or proliferation-related events, conditions or substances.

As used herein, the control of an angiogenic-, or proliferation-related event, condition, or substance refers to any qualitative or quantitative change in any type of factor, condition, activity, indicator, chemical or combination of chemicals, mRNA, receptor, marker, mediator, protein, transcriptional activity or the like, that may be or is believed to be related to angiogenesis or proliferation, and that results from administering the composition of the present invention. Those skilled in the art will appreciate that the invention extends to other compositions or compounds in the claims below, having the described characteristics. These characteristics can be determined for each test compound using the assays detailed below and elsewhere in the literature.

Other such assays include counting of cells in tissue culture plates or assessment of cell number through metabolic assays or incorporation into DNA of labeled (radiochemically, for example <3>H-thymidine, or fluorescently labeled) or immunoreactive (BrdU) nucleotides. In addition, antiangiogenic activity may be evaluated through endothelial cell migration, endothelial cell tubule formation, or vessel outgrowth in ex-vivo models such as rat aortic rings.

When administered orally, ITPP exhibits anti-tumor and antiproliferative activity with little or no toxicity. ITPP was tested for its ability to induce a decrease of the O₂-affinity of hemoglobin measured as a shift of the P50 value (P50 at 50% saturation of hemoglobin). With murine hemoglobin and whole blood, P50 shifts to higher PO₂ of up to 250% with hemoglobin and up to 40% with whole blood were observed.

The results obtained with ITPP in mice and pigs strongly suggest the possibility of its development as a therapeutic, due to its ability to enhance, in a regulated manner, oxygen delivery by red blood cells in the cases of blood flow impairment.

ITPP, when administered orally, intravenously, or intraperitoneally, inhibits angiogenesis in growing tumors by enhancing PO₂ in the forming tumors. Disclosed are further methods of regulation of vascular endothelial growth factor (VEGF) in a human or animal, by administering to the human or animal an effective amount of ITPP. More particularly, this disclosure provides for dose- dependent effects of ITPP on VEGF mRNA and protein expressions in the LLC cell line. VEGF gene expression in tumor bearing C57BL/6 mice was assayed and the effects of ITPP-induced down regulation of VEGF have been determined and correlated with modulation of cell proliferation. This disclosure resulted in the development of methods to control VEGF mRNA expression, protein concentration, and tumor cell proliferation. The results of these studies indicate a strong correlation between dose-dependent ITPP-induced down regulation of VEGF and cellular proliferation and suggests that ITPP can reduce VEGF mediated tumor angiogenesis, as well as the rate of tumor cell proliferation. Thus, down-regulation of VEGF by ITPP decreases tumor cell proliferation.

The shifting of the P50 value to higher O₂-partial pressures inhibits the expression of the hypoxia gene encoding VEGF in the tumors. Expression of the hypoxia gene encoding VEGF is necessary for angiogenesis to be stimulated in tumors. If this does not occur, angiogenesis is seriously inhibited and new vessels are not formed in tumors.

The results obtained concerning VEGF expression suggests that oxygen partial pressure in tumors is elevated upon administration of ITPP, as this elevation is the cause of inhibition of expression of this hypoxia gene. This observation raises a very important question, namely whether this enhancement of PO₂ may not act as a powerful radiosensitizer of cancer cells. Oxygen is a very potent radiosensitizer and, if indeed PO₂ in the tumors is enhanced by ITPP, this may have major consequences in enhancing the efficacy of radiation therapy of cancer.

ITPP is a potential significant adjuvant in the therapy of solid tumors as inhibitor of angiogenesis on one hand, and as radiosensitizer on the other.

Also contemplated by the present invention are implants or other devices comprised of the compounds or drugs of ITPP or prodrugs thereof where the drug or prodrug is formulated in a biodegradable or non-biodegradable polymer for sustained release. Non-biodegradable polymers release the drug in a controlled fashion through physical or mechanical processes without the polymer itself being degraded. Biodegradable polymers are designed to gradually be hydrolyzed or solubilized by natural processes in the body, allowing gradual release of the admixed drug or prodrug. The drug or prodrug can be chemically linked to the polymer or can be incorporated into the polymer by admixture. Both biodegradable and nonbiodegradable polymers and the process by which drugs are incorporated into the polymers for controlled release are well known to those skilled in the art. Examples of such polymers can be found in many references, such as Brem et al., J. Neurosurg 74: pp. 441-446 (1991). These implants or devices can be implanted in the vicinity where delivery is desired, for example, at the site of a tumor.

In addition to the compounds of the present invention, the pharmaceutical composition of this invention may also contain, or be co-administered (simultaneously or sequentially) with, one or more pharmacological agents of value in treating one or more disease conditions referred to hereinabove.

A person skilled in the art will be able by reference to standard texts, such as Remington's Pharmaceutical Sciences 17th edition, to determine how the formulations are to be made and how these may be administered.

Also disclosed is the use of compounds of ITPP or prodrugs thereof according to the present invention for the preparation of a medicament for the prophylaxis or treatment of conditions associated with angiogenesis or accelerated cell division or inflammation.

In a further aspect of the present invention there is provided a pharmaceutical composition comprising compounds of ITPP together with a pharmaceutically acceptable carrier, diluent or excipient.

The pharmaceutical composition may be used for the prophylaxis or treatment of conditions associated with angiogenesis or accelerated cell division or inflammation.

Also disclosed is a method of prophylaxis or treatment of a condition associated with angiogenesis or accelerated or increased amounts of cell division hypertrophic growth or inflammation, said method including administering to a patient in need of such prophylaxis or treatment an effective amount of compounds of ITPP or prodrugs thereof according to the present invention, as described herein. It should be understood that prophylaxis or treatment of said condition includes amelioration of said condition.

By "an effective amount" is meant a therapeutically or prophylactically effective amount. Such amounts can be readily determined by an appropriately skilled person, taking into account the condition to be treated, the route of administration and other relevant factors. Such a person will readily be able to determine a suitable dose, mode and frequency of administration.

The compositions described above can be provided as physiologically acceptable formulations using known techniques, and these formulations can be administered by standard routes. In general, the combinations may be administered by the topical, oral, rectal, intraperitoneal or parenteral (e.g., intravenous, subcutaneous or intramuscular) route. In addition, the combinations may be incorporated into polymers allowing for sustained release, the polymers being implanted in the vicinity of where delivery is desired, for example, at the site of a tumor. The dosage of the composition will depend on the condition being treated, the particular derivative used, and other clinical factors such as weight and condition of the patient and the route of administration of the compound. However, for oral administration, a recommended dosage is in the range of 0.1 to 5.0 g/kg/day. A preferred dosage for oral administration is in the range of 0.5 to 2.0 g/kg/day. An especially preferred dosage for oral administration is in the range of 0.80 to 1.0 g/kg/day.

The formulations in accordance with the present invention can be administered in the form of tablet, a capsule, a lozenge, a cachet, a solution, a suspension, an emulsion, a powder, an aerosol, a suppository, a spray, a pastille, an ointment, a cream, a paste, a foam, a gel, a tampon, a pessary, a granule, a bolus, a mouthwash, or a transdermal patch.

The formulations include those suitable for oral, rectal, nasal, inhalation, topical (including dermal, transdermal, buccal and sublingual), vaginal, parenteral (including subcutaneous, intramuscular, intravenous, intraperitoneal, intradermal, intraocular, intratracheal, and epidural) or inhalation administration. The formulations may conveniently be presented in unit dosage form and may be prepared by conventional pharmaceutical techniques. Such techniques include the step of bringing into association the active ingredient and a pharmaceutical carrier(s) or excipient(s). In general, the formulations are prepared by uniformly and intimately bringing into association the active ingredient with liquid carriers or finely divided solid carriers or both, and then, if necessary, shaping the product.

Formulations of the present invention suitable for oral administration may be presented as discrete units such as capsules, cachets or tablets each containing a predetermined amount of the active ingredient; as a powder or granules; as a solution or a suspension in an aqueous liquid or a non-aqueous liquid; or as an oil-in-water liquid emulsion or a water-in-oil emulsion, etc.

A tablet may be made by compression or molding, optionally with one or more accessory ingredients. Compressed tablets may be prepared by compressing, in a suitable machine, the active ingredient in a free-flowing form such as a powder or granules, optionally mixed with a binder, lubricant, inert diluent, preservative, surface-active or dispersing agent. Molded tablets may be made by molding, in a suitable machine, a mixture of the powdered compound moistened with an inert liquid diluent. The tablets may optionally be coated or scored and may be formulated so as to provide a slow or controlled release of the active ingredient therein.

Formulations suitable for topical administration in the mouth include lozenges comprising the ingredients in a flavored basis, usually sucrose and acacia or tragacanth; pastilles comprising the active ingredient in an inert basis such as gelatin and glycerin, or sucrose and acacia; and mouthwashes comprising the ingredient to be administered in a suitable liquid carrier.

Formulations suitable for topical administration to the skin may be presented as ointments, creams, gels and pastes comprising the ingredient to be administered in a pharmaceutical acceptable carrier. A preferred topical delivery system is a transdermal patch containing the ingredient to be administered.

Formulations for rectal administration may be presented as a suppository with a suitable base comprising, for example, cocoa butter or a salicylate.

Formulations suitable for nasal administration, wherein the carrier is a solid, include a coarse powder having a particle size, for example, in the range of 20 to 500 microns which is administered in the manner in which snuff is taken; *i.e.,* by rapid inhalation through the nasal passage from a container of the powder held close up to the nose. Suitable formulations, wherein the carrier is a liquid, for administration, as for example, a nasal spray or as nasal drops, include aqueous or oily solutions of the active ingredient.

Formulations suitable for vaginal administration may be presented as pessaries, tampons, creams, gels, pastes, foams or spray formulations containing, in addition to the active ingredient, ingredients such as carriers as are known in the art to be appropriate.

Formulation suitable for inhalation may be presented as mists, dusts, powders or spray formulations containing, in addition to the active ingredient, ingredients such as carriers as are known in the art to be appropriate.

Formulations suitable for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose containers, for example, sealed ampules and vials, and may be stored in freeze-dried (lyophilized) conditions requiring only the addition of a sterile liquid carrier, for example, water for injections, immediately prior to use. Extemporaneous injection solutions and suspensions may be prepared from sterile powders, granules and tablets of the kinds previously described.

Preferred unit dosage formulations are those containing a daily dose or unit, daily sub-dose, as herein above recited, or an appropriate fraction thereof, of the administered ingredient.

It should be understood that in addition to the ingredients, particularly mentioned above, the formulations of the present invention may include other agents conventional in the art having regard to the type of formulation in question, for example, those suitable for oral administration may include flavoring agents.

For the *in vitro* experiments ITPP was dissolved in deionized water, pH was adjusted at pH 7 and, for incubation with whole blood, the osmolality of the ITPP solutions was adjusted with glucose to 270-297 mOsM. Mixtures of hemoglobin and ITPP were measured with a HEMOX analyzer (PD Marketing, London) immediately. Red blood cells were incubated with ITPP for 1 hour at 37[deg.]C. Following incubation, the cells were washed 3 times with Bis-Tris-buffer (PH=7.0) and then used for P50 measurement.

In experiments conducted *in vivo,* in which ITPP was administered orally, a significant shift of the P50 value of circulating RBCs was observed. ITPP was dissolved in drinking water at a 20g/L-concentration (= 27mM, pH ~7.0.) and offered for drinking ad libitum.

The following examples illustrate the invention.

### EXAMPLE 1

### Induced Right Shift of the O₂-Hemoglobin Dissociation

### Curve (ODC) in Mice (Orally Administered)

Twelve (12) C57BL/6 mice were fed the ITPP-solution (20g/L-concentration = 27mM, pH -7.0) for 4 days (up to 25 ml per 24 hrs). Three (3) control mice drank pure water, and four (4) control mice a solution of myoinositol hexaphosphate (IHP) (same concentration and pH as ITPP). Blood was collected from all mice on day 0 (before treatment started), and on days 1, 2, 4, 6, 7, 8, 10, 11 and 12 (after treatment had started), in order to measure P50 values.

### Results

ITPP was neither rejected by the mice, nor harmful to the animals.

Oral application of ITPP caused significant right shifts of P50 (up to 31%) in mice.

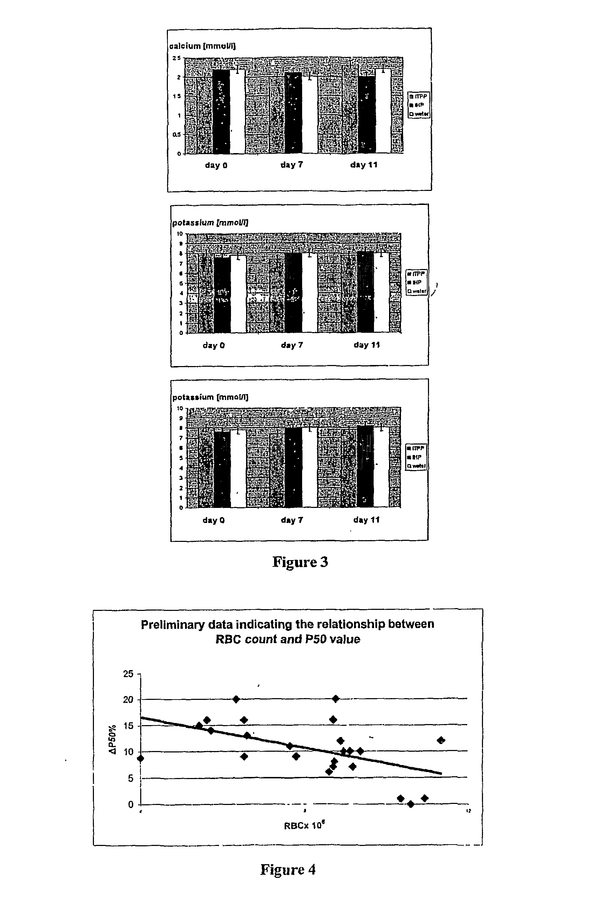

ITPP, when orally administered at a concentration of 27 mM, causes a right shift of the P50 value in murine circulating red blood cells (Figure 2). There is a time lag of approximately 48 hrs before the maximum shift is attained. Maximal P50 shifts are reached between day 2 and day 4, after beginning oral administration of ITPP. After 12 days, P50 values are back to control values, when ingestion is stopped on day 4. There is a significant effect of ITPP ingestion on the number of red blood cells. Although not wishing to be bound by theory, it is believed that effect of ITPP ingestion on the number of red blood cells (down-regulation of erythropoiesis) is due to the increased P₅₀. Hemolysis can be ruled out, as lysis of the red blood cells never occurred *in vitro.* The level of ions, such as sodium and potassium and calcium were normal after oral application of ITPP in mice (Figure 3). Figure 3 contains the mean values and SD for the serum concentration of sodium, potassium and calcium obtained on day 0, 7 and 11 after start drinking ITPP (4 mice), IHP (3 mice) or water (3 mice).

Blood counts were measured from all mice, on day 0, 7 and 11. The number of red blood cells in mice having ingested ITPP was reduced. There were no significant differences in the number of white blood cells (e.g. granulocytes, macrophages etc.) in blood from mice in different groups. Figure 4 shows the RBC counts for mice with shifted ODC as compared to controls. Figure 4 further shows the relation of P₅₀ shift [%] to number of erythrocytes/mm³ in mice having received ITPP. It appears, based upon preliminary data, that an inverse relationship exists between the number of red blood cells and shift of their P₅₀ value. The basal value of the red blood cell count is restored, once ΔP₅₀ becomes 0%, 12 days after ingestion of ITPP.

### EXAMPLE 2

### Induced Right Shift of the ODC in Mice (Injected Intraperitoneally)

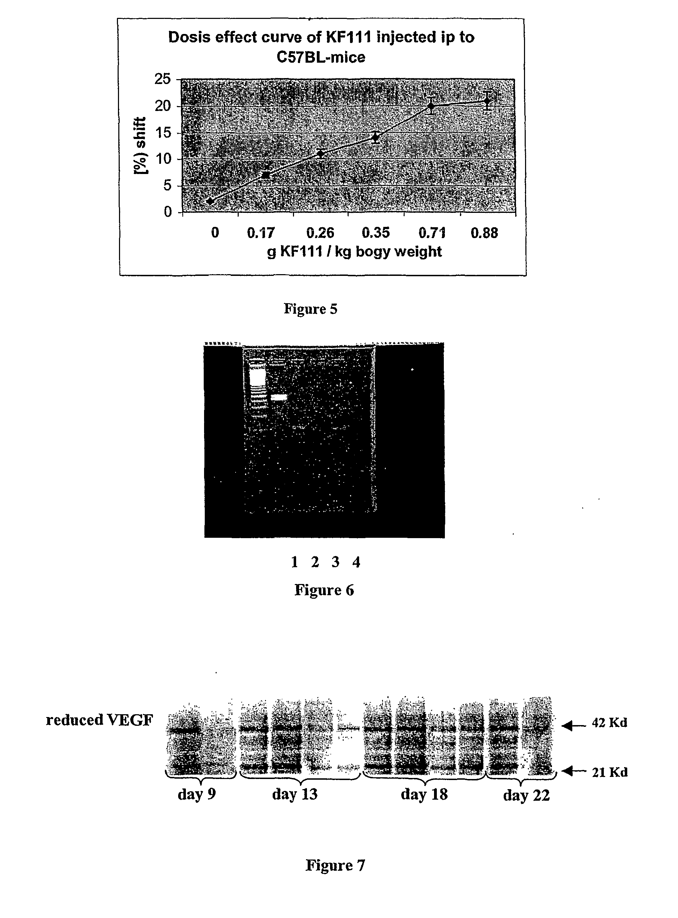

When ITPP (pH 7, 200µl) was injected intraperitoneally in mice, the P₅₀ values of circulating red blood cells were shifted up to 23%. Figure 5 demonstrates that ITPP was well tolerated by mice, up to a concentration of 150mM. The level of ions, such as sodium, potassium and calcium were normal after ip injection. Six (6) mice were each injected intraperitoneally with 45-150 mM (= 0.17-0.88 g/kg body weight) of ITPP. Means of % shift and standard deviation are shown in Figure 5.

The concentration dependence of the P₅₀ shifts induced by ITPP is an additional indication that this compound crosses the plasma membrane of the red blood cells.

### EXAMPLE 3

### Induced Right Shift of the OCD in Piglets (Intravenously Injected)

ITPP was also injected intravenously (IV) in piglets. A right shift of P₅₀ was observed, when the compound was injected at a 19/kg body weight dose.

In order to check possible side effects of ITPP the level of calcium in the serum of the injected piglet was determined. A strong drop in the Ca²⁺ concentration in the animal's blood immediately after infusion indicated the possibility that ITPP, with 3 dissociated phosphate groups binds Ca²⁺, reducing thus its availability as free ion in the blood. One day after infusion the concentration of Ca²⁺ in the piglets' blood was restored to the normal value. The results are shown in Table 1.

**Table 1: Ca²⁺ concentration in the piglet's circulation blood**

| | Ca²⁺ conc. |
|---|---|
| Sample taken | [mmol/L] |
| Before injection | 2.38 |
| 10 min after completion of injection | 1.73 |
| 24 hrs after injection | 2.36 |

Based upon this observation, a CaCl₂ (equimolar to ITPP) solution was injected with the ITPP solution, so that the dissociated phosphate groups of ITPP were saturated. None of the side effects observed previously occurred. The level of calcium remained constant and the P₅₀ shift was again approximately 20% of the basal value. The level of the other ions sodium and potassium was unchanged after IV injection of ITPP in piglets.

### EXAMPLE 4

### Effect of in vivo Lowering of Hemoglobin's Affinity for O₂ by ITPP on Intratumoral PO₂, Angiogenesis and Expression of VEGF mRNA

ITPP, when administered orally, intravenously, or intraperitoneally, inhibits angiogenesis in growing tumors by enhancing PO₂ in the forming tumors. Thirty (30) C57BL/6 mice received 20g/L of ITPP orally until the P₅₀ value showed a shift of at least 20% above the control value. All animals received then 1x10⁶ Lewis Lung carcinoma (LLC) cells, injected in the dorsal cavity. At different time points, the VEGF mRNA were assayed by RT-PCR in the tumors growing in both groups of mice.

Tumor tissue samples were ground in a RIPA lysis buffer (1% Nonidet p-40 detergent, 50 mM Tris pH 8.0, 137 mM NaCl, 10% glycerol) supplemented with protease inhibitor cocktail (Roche, Reinach, Switzerland). After centrifugation (10 minutes, 4°C and 12,000 g), protein concentrations of tissue extracts were determined according to the Bradford method. Detergent soluble protein samples (10 mg) were size separated by SDS-PAGE in 10% acrylamide gels and transferred to nitrocellulose membrane (Protran BA 85, Schleicher and Schuell, Dassel, Germany). Membranes were blocked for 3 hours at room temperature in 10% skim milk in Tris buffer saline containing 0.1 % Tween, before an overnight incubation at 4°C with rabbit polyclonal antibodies recognizing human, mouse and rat vascular endothelial growth factor (VEGF A-20, sc-152, Santa Cruz Biotechnology, Santa Cruz, California) at a dilution of 1:200. Membranes were then probed for primary antibody with anti-rabbit (1:16,000) peroxidase conjugates (Sigma-Aldrich, L'Isle d'Abeau Chesnes, France) for 60 minutes at room temperature. The resulting complexes were visualized by enhanced chemiluminescence autoradiography (Amersham Pharma Biotech, Orsay, France).

There was a difference in the level of mRNA of the VEGF gene in both groups. Figure 6 shows an agarose gel indicating the VEGF mRNA concentrations in tumors from control and ITPP drinking animals. The RT-PCR agarose gel assay of VEGF mRNAs from tumor tissue taken from 2 mice each on day 15 after inoculation of LLC cells (track 1: controls, track 2: ITPP treated animals) and day 30 after inoculation (track 3: control animals, track 4: ITPP treated animals). Figure 7 shows the Western blot assay of the expressed VEGF in tumors of control and ITPP-treated LLC tumor-bearing animals.

Quantification of the gel assays indicated a reduction by a factor of 10,000 of the amount of VEGF mRNAs detected in the tumors of animals having received ITPP, at day 9 and then, while differences remain between treated and untreated animals, they tend to decrease. This indicates that ITPP taken up by circulating red blood cells significantly increases tumor PO₂.

### REFERENCES

1. Fylaktakidou, K., Lehn, J.-M., Greferath, R., and Nicolau, C. (2004) Bioorg.Med.Chem. Lett (submitted)
2. Kim KJ, Li B, Winer J, Armanini M, Gillett N, Phillips HS, Ferrara N (1993) Nature 362, 841-844.
3. Kandel J, Bossy-Wetzel E, Radvanyi F, Klagsbrun M, Folkman J, Hanahan D (1991) Cell 66, 1095-1104.
4. O'Reilly MS, Boehm T, Shing Y, Fukai N, Vasios G, Lane WS, Flynn E, Birkhead JR, Olsen BR, Folkman J (1997) Cell 88, 277-285.
5. Good DJ, Polverini PJ, Rastinejad F, Le Beau MM, Lemons RS, Frazier WA, Bouck NP. (1990) Proc Natl Acad Sci USA 87, 6624-6628.
6. O'Reilly MS, Holmgren L, Shing Y, Chen C, Rosenthal RA, Moses M, Lane WS, Cao Y, Sage EH, Folkman J (1994) Cell 79, 315-328.
7. Chen C, Parangi S, Tolentino MJ, Folkman J. (1995) Cancer Res. 55, 4230-4233.
8. Ferrara N. (2002) Nat. Rev. Cancer 2, 795-803.
9. Ferrara N, Davis-Smyth T (1997) Endocr Rev. 18, 4-25.
10. Ferrara N, Gerber HP, LeCouter J. (2003) Nat Med. 9, 669-676.
11. Fontanini G, Vignati S, Boldrini L, Chine S, Silvestri V, Lucchi M, Mussi A, Angeletti CA, Bevilacqua G. (1997) Clin Cancer Res. 3, 861-865.
12. Dor Y, Porat R, Keshet E. (2001) Am J Physiol Cell Physiol. 280, Cl 367-1374.
13. Brizel DM, Scully SP, Harrelson JM, Layfield LJ, Bean JM, Prosnitz LR, Dewhirst MW (1996) Cancer Res. 56, 941-943.

## Claims

1. Inositol-trispyrophosphate or salts thereof for use in the therapy of blood borne tumors, solid tumors and tumor metastasis in a human or animal with cancer, wherein the tumors are associated with abnormal angiogenesis.

2. Salt of Inositol-trispyrophosphate for use as claimed in claim 1, wherein the salt of inositol-trispyrophosphate is a Na, Ca, or NH₄ salt of inositol-trispyrophosphate.

3. Inositol-trispyrophosphate or salts thereof for the use of claim 1, wherein the inositol-trispyrophosphate is in a daily dose, a daily sub-dose, or any appropriate fraction thereof, to the human or animal to inhibit abnormal angiogenesis.

4. Inositol-trispyrophosphate or salts thereof for the use of claim 1, wherein the tumor expresses VEGF.

5. Inositol-trispyrophosphate or salts thereof for the use of claim 1, wherein the cancer is selected from breast cancer, prostate cancer, renal cell cancer, brain cancer, ovarian cancer, colon cancer, bladder cancer, pancreatic cancer, stomach cancer, esophageal cancer, cutaneous melanoma, liver cancer, lung cancer, testicular cancer, kidney cancer, bladder cancer, cervical cancer, lymphoma, parathyroid cancer, penile cancer, rectal cancer, small intestine cancer, thyroid cancer, uterine cancer, Hodgkin's lymphoma, lip and oral cancer, skin cancer, leukemia or multiple myeloma.

6. Inositol-trispyrophosphate or salts thereof for the use of claim 1, wherein the tumor is an hypoxic tumor.

7. Inositol-trispyrophosphate or salts thereof for the use of claim 1, wherein the inositol-trispyrophosphate is for oral, topical, rectal, parenteral, or intraperitoneal administration.

8. Inositol-trispyrophosphate or salts thereof for the use of claim 1, wherein the inositol-trispyrophosphate is for intravenous, subcutaneous or intramuscular administration.

9. Inositol-trispyrophosphate or salts thereof for the use of claim 7, wherein the inositol-trispyrophosphate is for administration in a composition comprising an additive selected from an anti-oxidant, a buffer, a bacteriostat, a liquid carrier, a solute, a suspending agent, a thickening agent, a flavoring agent, a gelatin, glycerin, a binder, a lubricant, an inert diluent, a preservative, a surface active agent, a dispersing agent, a biodegradable polymer, or any combination thereof.

10. Inositol-trispyrophosphate or salts thereof for the use of claim 1, wherein the inositol-trispyrophosphate is for administration in the form of tablet, a capsule, a lozenge, a cachet, a solution, a suspension, an emulsion, a powder, an aerosol, a suppository, a spray, a pastille, an ointment, a cream, a paste, a foam, a gel, a tampon, a pessary, a granule, a bolus, a mouthwash, or a transdermal patch.

11. Inositol-trispyrophosphate or salts thereof for the use of claim 1, wherein the inositol-trispyrophosphate is formulated in a biodegradable or non-biodegradable polymer for sustained release, or in unit-dose or multi-dose containers.

12. Inositol-trispyrophosphate or salts thereof for the use of claim 1, wherein the inositol-trispyrophosphate is stored in freeze-dried conditions requiring the addition of a sterile liquid carrier immediately prior to use.

13. Inositol-trispyrophosphate or salts thereof for the use of any one of preceding claims, wherein the inositol-trispyrophosphate is administered by intravenous injection with an equimolar solution of calcium chloride.

## Patentansprüche

1. Inositoltrispyrophosphat oder Salze davon zur Verwendung in der Therapie von blutgetragenen Tumoren, festen Tumoren und Tumormetastasen in einem Menschen oder Tier mit Krebs, wobei die Tumoren mit abnormaler Angiogenese assoziiert sind.

2. Salz von Inositoltrispyrophosphat zur Verwendung wie in Anspruch 1 beansprucht, wobei das Salz von Inositoltrispyrophosphat ein Na-, Ca- oder NH₄-Salz von Inositoltrispyrophosphat ist.

3. Inositoltrispyrophosphat oder Salze davon zur Verwendung von Anspruch 1, wobei das Inositoltrispyrophosphat in einer täglichen Dosis, einer täglichen Unterdosis oder jeglichem geeigneten Teil davon an den Menschen oder das Tier ist, um abnormale Angiogenese zu hemmen.

4. Inositoltrispyrophosphat oder Salze davon zur Verwendung von Anspruch 1, wobei der Tumor VEGF exprimiert.

5. Inositoltrispyrophosphat oder Salze davon zur Verwendung von Anspruch 1, wobei der Krebs ausgewählt ist aus Brustkrebs, Prostatakrebs, Nierenzellkrebs, Gehirnkrebs, Eierstockkrebs, Kolonkrebs, Blasenkrebs, Bauchspeicheldrüsenkrebs, Magenkrebs, Speiseröhrenkrebs, kutanem Melanom, Leberkrebs, Lungenkrebs, Hodenkrebs, Nierenkrebs, Blasenkrebs, Gebärmutterhalskrebs, Lymphom, Nebenschilddrüsenkrebs, Peniskrebs, Enddarmkrebs, Dünndarmkrebs, Schilddrüsenkrebs, Gebärmutterkrebs, Hodgkins Lymphom, Lippen- und Mundkrebs, Hautkrebs, Leukämie oder multiplem Myelom.

6. Inositoltrispyrophosphat oder Salze davon zur Verwendung von Anspruch 1, wobei der Tumor ein hypoxischer Tumor ist.

7. Inositoltrispyrophosphat oder Salze davon zur Verwendung von Anspruch 1, wobei das Inositoltrispyrophosphat zur oralen, topischen, rektalen, parenteralen oder intraperitonealen Verabreichung ist.

8. Inositoltrispyrophosphat oder Salze davon zur Verwendung von Anspruch 1, wobei das Inositoltrispyrophosphat zur intravenösen, subkutanen oder intramuskulären Verabreichung ist.

9. Inositoltrispyrophosphat oder Salze davon zur Verwendung von Anspruch 7, wobei das Inositoltrispyrophosphat zur Verabreichung in einer Zusammensetzung umfassend einen Zusatzstoff ausgewählt aus einem Antioxidans, einem Puffer, einem Bakteriostatikum, einem flüssigen Träger, einem Solut, einem Suspensionsmittel, einem Verdickungsmittel, einem Aromastoff, einer Gelatine, Glycerin, einem Bindemittel, einem Schmiermittel, einem inerten Verdünnungsmittel, einem Konservierungsmittel, einem oberflächenaktiven Agens, einem Dispersionsmittel, einem biologisch abbaubaren Polymer oder jeglicher Kombination davon ist.

10. Inositoltrispyrophosphat oder Salze davon zur Verwendung von Anspruch 1, wobei das Inositoltrispyrophosphat zur Verabreichung in Form von Tablette, einer Kapsel, einer Lutschtablette, einem Cachet, einer Lösung, einer Suspension, einer Emulsion, eines Pulvers, eines Aerosols, eines Zäpfchens, eines Sprays, einer Pastille, einer Salbe, einer Creme, einer Paste, eines Schaums, eines Gels, eines Tampons, eines Pessars, eines Granulats, eines Bolus, einer Mundspülung oder eines transdermalen Pflasters ist.

11. Inositoltrispyrophosphat oder Salze davon zur Verwendung von Anspruch 1, wobei das Inositoltrispyrophosphat in einem biologisch abbaubaren oder nicht biologisch abbaubaren Polymer zur verzögerten Freisetzung oder in Einheitsdosis- oder Mehrdosis-Behältern formuliert ist.

12. Inositoltrispyrophosphat oder Salze davon zur Verwendung von Anspruch 1, wobei das Inositoltrispyrophosphat unter gefriergetrockneten Bedingungen gelagert wird, die die Zugabe eines sterilen flüssigen Trägers unmittelbar vor Benutzung erfordern.

13. Inositoltrispyrophosphat oder Salze davon zur Verwendung von irgendeinem der vorhergehenden Ansprüche, wobei das Inositoltrispyrophosphat durch intravenöse Injektion mit einer äquimolaren Lösung von Calciumchlorid verabreicht wird.

## Revendications

1. Trispyrophosphate d'inositol ou ses sels pour une utilisation dans le traitement de tumeurs hématologiques, de tumeurs solides et de métastases tumorales chez un être humain ou un animal souffrant d'un cancer, les tumeurs étant associées à une angiogenèse anormale.

2. Sel de trispyrophosphate d'inositol pour une utilisation telle que revendiquée dans la revendication 1, le sel de trispyrophosphate d'inositol étant un sel de Na, Ca, ou NH₄ du trispyrophosphate d'inositol.

3. Trispyrophosphate d'inositol ou ses sels pour l'utilisation selon la revendication 1, où le trispyrophosphate d'inositol est administré dans une dose quotidienne, une sous-dose quotidienne, ou toute fraction appropriée de celle-ci, à l'être humain ou à l'animal pour inhiber une angiogenèse anormale.

4. Trispyrophosphate d'inositol ou ses sels pour l'utilisation selon la revendication 1, où la tumeur exprime le VEGF.

5. Trispyrophosphate d'inositol ou ses sels pour l'utilisation selon la revendication 1, où le cancer est choisi parmi le cancer du sein, le cancer de la prostate, le cancer des cellules rénales, le cancer du cerveau, le cancer ovarien, le cancer du côlon, le cancer de la vessie, le cancer pancréatique, le cancer de l'estomac, le cancer oesophagien, le mélanome cutané, le cancer du foie, le cancer du poumon, le cancer testiculaire, le cancer du rein, le cancer de la vessie, le cancer du col de l'utérus, le lymphome, le cancer de la parathyroïde, le cancer pénien, le cancer rectal, le cancer de l'intestin grêle, le cancer de la thyroïde, le cancer de l'utérus, le lymphome de Hodgkin, le cancer de la lèvre et buccal, le cancer de la peau, la leucémie ou le myélome multiple.

6. Trispyrophosphate d'inositol ou ses sels pour l'utilisation selon la revendication 1, où la tumeur est une tumeur hypoxique.

7. Trispyrophosphate d'inositol ou ses sels pour l'utilisation selon la revendication 1, où le trispyrophosphate d'inositol est destiné à une administration orale, topique, rectale, parentérale, ou intrapéritonéale.

8. Trispyrophosphate d'inositol ou ses sels pour l'utilisation selon la revendication 1, où le trispyrophosphate d'inositol est destiné à une administration intraveineuse, sous-cutanée ou intramusculaire.

9. Trispyrophosphate d'inositol ou ses sels pour l'utilisation selon la revendication 7, où le trispyrophosphate d'inositol est destiné à une administration dans une composition comprenant un additif choisi parmi un antioxydant, un tampon, un bactériostatique, un support liquide, un soluté, un agent de suspension, un agent épaississant, un agent aromatisant, une gélatine, la glycérine, un liant, un lubrifiant, un diluant inerte, un conservateur, un agent tensioactif, un agent dispersant, un polymère biodégradable, ou l'une quelconque de leurs combinaisons.

10. Trispyrophosphate d'inositol ou ses sels pour l'utilisation selon la revendication 1, où le trispyrophosphate d'inositol est destiné à une administration sous la forme d'un comprimé, une capsule, une tablette, un cachet, une solution, une suspension, une émulsion, une poudre, un aérosol, un suppositoire, une pulvérisation, une pastille, une pommade, une crème, une pâte, une mousse, un gel, un tampon, un ovule, un granulé, un bolus, un bain de bouche, ou un patch transdermique.

11. Trispyrophosphate d'inositol ou ses sels pour l'utilisation selon la revendication 1, où le trispyrophosphate d'inositol est formulé dans un polymère biodégradable ou non biodégradable pour une libération prolongée, ou dans des récipients à dose unitaire ou multidoses.

12. Trispyrophosphate d'inositol ou ses sels pour l'utilisation selon la revendication 1, où le trispyrophosphate d'inositol est stocké dans des conditions cryodesséchées nécessitant l'addition d'un support liquide stérile immédiatement avant usage.

13. Trispyrophosphate d'inositol ou ses sels pour l'utilisation selon l'une quelconque des revendications précédentes, où le trispyrophosphate d'inositol est administré par injection intraveineuse avec une solution équimolaire de chlorure de calcium.
